# EUROPEAN PATENT SPECIFICATION

(11) **EP 2 231 205 B1**
(45) Date of publication and mention of the grant of the patent: **08.04.2015**
(21) Application number: 09702854.2
(22) Date of filing: 18.01.2009
(51) Int. Cl.: A61K 35/60, A61K 38/39, A61L 15/32, A61L 15/40

(54) **COLLOIDAL COLLAGEN BURN WOUND DRESSING PRODUCED FROM JELLYFISH**
AUS QUALLEN HERGESTELLTER KOLLOIDAL-COLLAGEN-VERBAND FÜR BRANDWUNDEN
PANSEMENT À BASE DE COLLAGÈNE COLLOÏDAL POUR BLESSURES PAR BRÛLURE PRODUIT À PARTIR DE MÉDUSE

(30) Priority: 16.01.2008 US 21381 P
(43) Date of publication of application: 29.09.2010
(73) Proprietor: Coll-Med Ltd, 23100 Migdal HaEmek (IL)
(72) Inventor: ANGEL, Samuel, 75245 Rishon-Lezion (IL)
(74) Representative: Stainthorpe, Vanessa Juliet
(86) International application number: PCT/IL2009/000071
(87) International publication number: WO 2009/090655

(56) References cited:
- WO-A1-95/17428
- WO-A1-99/63964
- US-A- 3 821 371
- US-A- 5 210 182
- US-A- 5 698 228
- US-A- 5 944 866
- US-A1- 2002 147 154
- US-A1- 2003 004 315
- US-A1- 2004 167 318
- US-A1- 2006 149 182
- US-A1- 2007 073 210
- US-B1- 6 541 023
- US-B1- 6 660 280

## Description

### FIELD OF THE INVENTION

The present invention is directed to burn wound dressings comprised of colloidal collagen gel produced from jellyfish.

More specifically, the present invention is directed to films and film/fabric composites and methods for preparation thereof, wherein the films or film/fabric composites are produced from jellyfish and are comprised of a colloidal collagen salt with sufficient viscosity to enable production of a stable gel for use as a burn wound dressing film, without the need for outside cross-linking agents.

### BACKGROUND

Collagen is used worldwide in cosmetics and pharmaceuticals. It is usually obtained from cattle hides, pork skins, or connective tissues from various animals, and it is usually extracted as soluble collagen. Collagen sources from animals carry with them the possibilities of infection with viral diseases such as BSE or Mad Cow Disease, Hoof and Mouth Disease, Hog Cholera, Avian Flu and others. The fastidious dehairing, blood and fat removal necessary from the skins of these animals also exposes the workers to infection from these above mentioned diseases, and results in relatively high costs.

Jellyfish blooms are increasing world wide and are thus becoming more and more of a nuisance and the cause of serious damage to the fishing industry. An object of the present invention is to help transfer a nuisance or potential source of damage into a useful and beneficial commodity. Jellyfish are comprised of approximately 97-98 % water and about 3% salt. Protein makes up most of the rest of the solids and the major part of that protein is collagen.

Collagen prepared from jellyfish is not subject to infection from animal diseases. Jellyfish are relatively easy to clean, and do not require dehairing, blood or fat removal, as in animal skins, nor do they depend on animal or fish skin removal and de-lipidation.

Collagen formation is an essential part of various phases of the healing process in serious 2^{nd}, 3^{rd} and 4^{th} degree burn wounds and wounds in general. Collagen is laid down in the human body and in tissue renewal in wounds by fibroblasts. The quicker and more effectively fibroblasts proliferate, the quicker collagen regenerative tissue can develop. Fibroblast proliferation is enhanced by adhesion to collagen scaffolds, particularly scaffolds including Types I, II, and/or III collagen. Collagen scaffolds constructed from jellyfish have shown superior human fibroblast cell proliferation and viability as compared to bovine collagen scaffolds (Collagen scaffolds derived from marine sources and their biocompatibility, Eun Song, So Yeon Kim et al. Biomaterials 27:29512961 2006). For all of the above reasons, it would be advantageous to have collagen films for wound healing comprised from jellyfish collagen. To date, collagen films from jellyfish tissue have only been produced using solubilized collagen gels, which are generally not stable and turn rubbery upon standing. They are also not thermally stable without the introduction of cross-linking agents, which can potentially have adverse effects for wound dressing applications. It is an object of the present invention to produce collagen films for wound healing from non-solubilized jellyfish collagen, which would not require introduction of cross-linking agents.

It has long been known that electrolytes, acting on proteins, form non-protein compounds. Proteins combine with acids and bases stoichiometrically which occurs only when the hydrogen ion concentration is above the isoelectric pH, known as the isoelectric point, of the protein. If a protein with a tendency to electrolytic dissociation is treated with an acid of the right concentration, a protein salt is formed. This results in a colloidal solution, having an increased viscosity due to the formation of aggregates out of isolated proteins or ions. The stability of the colloidal solution is based on the electrical potential difference between the colloidal particles (micellae) and the surrounding liquid. Addition of too much acid or base causes increased electrical potential difference between the particles and the liquid and thus, decreased stability.

In the 1960s and 1970s, several US and Canadian patents were issued to Battista and others, in which it was disclosed that using a particular concentration of HCl with cattle skin collagen, with strong agitation, it was possible to obtain viscous collagen salts, the viscosity of which remained stable over a period of several weeks. These patents include Canadian Patent Number 806621, entitled "Coatings of microcrystalline collagen", Canadian Patent Number 814301, entitled "Colloidal compositions and methods"; Canadian Patent Number 856216, entitled "Foods, pharmaceuticals and cosmetics containing a salt of collagen", US Patent Number 3,628,974 entitled, "Microcrystalline collagen, an ionizable partial salt of collagen and foods, pharmaceuticals and cosmetics containing same", US Patent Number 3,742,955, entitled, "Fibrous collagen derived product having hemostatic and wound binding properties", Canadian Patent Number 953054, entitled "Method of forming structures from microcrystalline collagen"; and Canadian Patent Number 964131 to Zeleznick, entitled "Microcrystalline collagen structures and method of preparing same."

All of the methods disclosed in the above-referenced patents disclose preparation of collagen-salts from animal collagen sources. However, preparation of collagen-salts from jellyfish sources has not been taught. Moreover, preparation of collagen-salts from jellyfish sources cannot be accomplished in the same manner as preparation of collagen-salts from animal sources, due to differences in collagen type, the nature of the tissue and other factors. To date, although it is clear that films produced from jellyfish collagen would provide enhanced fibroblast proliferation and thus enhanced wound healing, there are no known wound dressing films produced from jellyfish collagen at least partially due to the lack of thermal stability in the absence of cross-linking agents.

It would therefore be advantageous to have a method of preparation of a collagen-salt gel bum wound film dressing from jellyfish, thus maximizing the benefits of jellyfish, while retaining a thermally stable collagen gel without the need for adding cross-linking agents to the process.

US 6541023 is directed to the use of fish skin to obtain collagen for the production of supports for tissue engineering.

WO 99/63964 is directed to a method for preparing fibrillin from cnidaria.

### SUMMARY OF THE INVENTION

The invention is defined in the appended claims. There is provided, in accordance with one embodiment of the present invention, a method of producing a collagen-based wound dressing. The method includes providing a jellyfish tissue, adding an acid to the jellyfish tissue to produce a collagen-salt solution, mixing the collagen-salt solution to form a viscous gel, and forming a burn dressing from the viscous gel. The wound dressing may be a film or a film/fabric composite.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of various embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several embodiments of the invention may be embodied in practice.

In the drawings:
FIG. 1 is a flowchart diagram of a method of producing a jellyfish collagen film in accordance with embodiments of the present invention;
FIG. 2A is a schematic illustration of a jellyfish collagen film, in accordance with an embodiment of the present invention; and
FIG. 2B is a schematic illustration of a jellyfish collagen film/fabric composite, in accordance with an embodiment of the present invention.

It will be appreciated that for simplicity and clarity of illustration, elements shown in the drawings have not necessarily been drawn accurately or to scale. For example, the dimensions of some of the elements may be exaggerated relative to other elements for clarity or several physical components may be included in one functional block or element. Further, where considered appropriate, reference numerals may be repeated among the drawings to indicate corresponding or analogous elements. Moreover, some of the blocks depicted in the drawings may be combined into a single function.

### DETAILED DESCRIPTION OF THE INVENTION

In the following detailed description, numerous specific details are set forth in order to provide a thorough understanding of the present invention. It will be understood by those of ordinary skill in the art that embodiments of the present invention may be practiced without these specific details. In other instances, well-known methods, procedures, components and structures may not have been described in detail so as not to obscure the present invention.

The present invention relates to a collagen film or film/fabric composite made from a collagen-salt gel, and methods of preparation thereof In embodiments of the present invention, a collagen film is formed from a gel or slurry of a colloidal form of collagen extracted from jellyfish tissue. Although the formation of a colloidal form of collagen extracted from bovine tissue is known, several factors complicate the process when applied to jellyfish. First, jellyfish tissue is much more aqueous than animal or fish skins. The solidity of bovine tissue, for example, allows for it to be freeze-dried and directly solubilized in acid. In contrast, the jellyfish tissue is highly aqueous, which makes the process of freeze-drying very time consuming and therefore expensive. In addition, some types of jellyfish, such as the Rhopilema nomadica jellyfish tissue used in the examples of the present application, are comprised primarily of types II and III collagen, whereas bovine tissue is comprised mostly of type I collagen. Type I collagen is more thermally stable than types II and III due to differences in amino acid composition, side chain composition, hydrophobic areas, etc. As such, the parameters for forming colloidal collagen from jellyfish must accommodate the relative thermal instability of the form of collagen found therein.

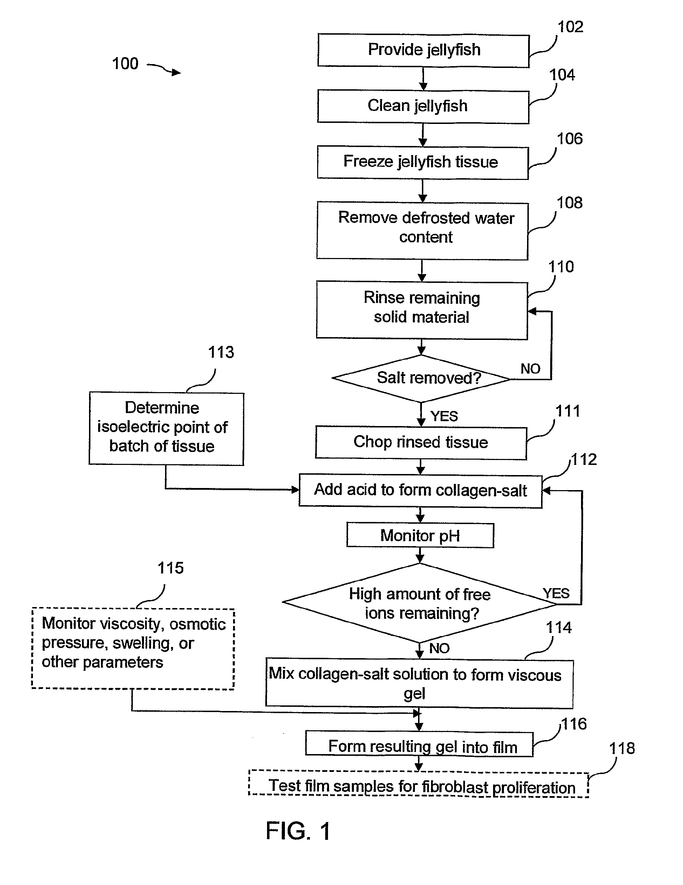

Reference is now made to FIG. 1, which is a flowchart diagram of a method 100 for forming a jellyfish collagen film, in accordance with embodiments of the present invention. First, a jellyfish is provided (step 102). In some embodiments, the jellyfish is a Rhopilema nomadica jellyfish, commonly found along the coast of Israel. The Rhopilema nomadica jellyfish has an umbrella made up of two cellular layers: the ectoderm and the endoderm. Between these two layers lies the mesoglea, a noncellular tissue with collagen fibers in a gelatinous substance. All three layers are used in the present invention, although embodiments of the invention may include use of only the mesoglea layer. In other embodiments, the jellyfish is any other type of jellyfish which may be suitable for formation of collagen films. The jellyfish is cleaned (step 104) by removing tentacles and washing to remove sand and mucous membranes from the umbrella's endoderm. Next, the cleaned jellyfish tissue is cut into pieces and immediately frozen (step 106) and stored, for example, in shallow containers for rapid freezing so as to avoid deterioration of the collagen in the jellyfish tissue. Next, an amount of frozen jellyfish tissue is defrosted, and the defrosted water content is removed (step 108) from the remaining "solid material". In this context "solid material" refers to the remaining block of tissue after defrosted water has been removed. However, a large volume of the "solid material" is generally comprised of water as well. It should be readily apparent that since the jellyfish tissue is frozen into blocks, a desired amount of frozen jellyfish tissue may be defrosted as needed. Removal of the defrosted water results in removal of some of the salt which is naturally present in the jellyfish tissue. The remaining solid material is then rinsed (step 110) in deionized ice water to remove the excess salt. Rinsing may be done several times in a high volume of water (1:10 ratio of solid material volume to water volume, for example) to ensure that the salt is sufficiently removed. The amount or concentration of starting fresh or frozen tissue used is approximately three times the amount as compared to cowhide for a similar yield of usable collagen gel. Generally, following desalting about 0.1% of the wet weight of the raw material will remain as dry weight. Of this, the collagen yield is approximately 90%.

Statistically representative aliquots of the solid desalted umbrella pieces are taken for dry weight and water content determination. The isoelectric points of representative samples of desalted skins are determined by isoelectric focusing and other methods for each batch of jellyfish. Batches may be caught at different seasons of the year and may have slight variations in isoelectric point and other parameters.

Next, the desalted, rinsed tissue is finely chopped (step 111), using, for example, a refrigerated bowl chopper maintained at approximately 0° C. Chopping may result in a paste-like consistency. The chopped material is put into a container or vessel, which may include a magnetic stirrer or an overhead stirring mechanism. An acid is added (step 112) in an amount to reach a predetermined acid concentration (which depends on the amount of water left in the material). The pre-determined acid concentration and normality will depend on the isoelectric point of the tissue, which is determined (step 113) per batch as described above. The amount of tissue in any given batch may vary depending on the availability of the particular jellyfish being used, but may be hundreds of kilograms worth or even more. This determination may be done well in advance of the step of adding an acid to form the colloidal collagen salt. It is known that if the pH of a protein is below but near the isoelectric point, the more acid added, the more non inorganic protein is transformed into a salt. Thus the object is to maximize as much as possible this transformation to a protein salt colloid and to obtain maximum viscosity without causing a depressing effect by an excess of acid. In this way, a protein salt obtainable by stoichiometric combination of the acid with the jellyfish solids may be maximized. With hydrochloric acid the amount added is adjusted to between a normality of between 0.001 and 0.1 depending on the isoelectric point of the jellyfish tissue, and with jellyfish tissue at a calculated dry solids content at a concentration between 1 and 3%,.

While the acid is added, the pH may be monitored so as to determine how much free ion (H⁺) remains and how much has been taken up by the protein to form a protein salt. This way, over-addition of the acid can be avoided. The collagen-salt solution is mixed, by stirring, blending or agitation, (step 114) to form a viscous gel. In one embodiment, this is done by grinding and forming a homogenate. In another embodiment, stirring may be done using a magnetic stirrer. Blending may be accomplished by using a blender type apparatus to sufficiently stir the acid with the chopped material. The use of more aggressive agitation using a blender may be required to obtain maximum protein salt formation. Agitation must be carried out without causing an excess of air pockets, which can potentially denature the collagen. The rate of stirring may be in a range of 1,000-10,000 rpm, for example, but is not limited to those speeds. In one embodiment, the temperature is adjusted to 0 centigrade and the entire apparatus is placed under vacuum. Slow agitation commences for about 10 minutes to allow thorough mixing of the acid with the umbrella tissue. Agitation is then increased and can reach 10,000 rpm. Temperature is monitored not to surpass 25 degrees centigrade during the mixing-blending operations. Agitation and blending can take between 10 to 30 minutes to obtain the optimum viscosity required for a film formation with desirable tensile strength properties. The viscosity of the gel and the thermal stability will vary based on jellyfish tissue concentration, acid pH, agitation speed, agitation time and other parameters. Thermal resistance to denaturation may be tested at different temperatures.

In some embodiments, during the process of grinding, agitation or stirring, measurements of viscosity, osmotic pressure, swelling or other parameters may be made (step 115) for monitoring purposes. Viscosity measurements may be taken using any standard viscometer at various intervals, for example.

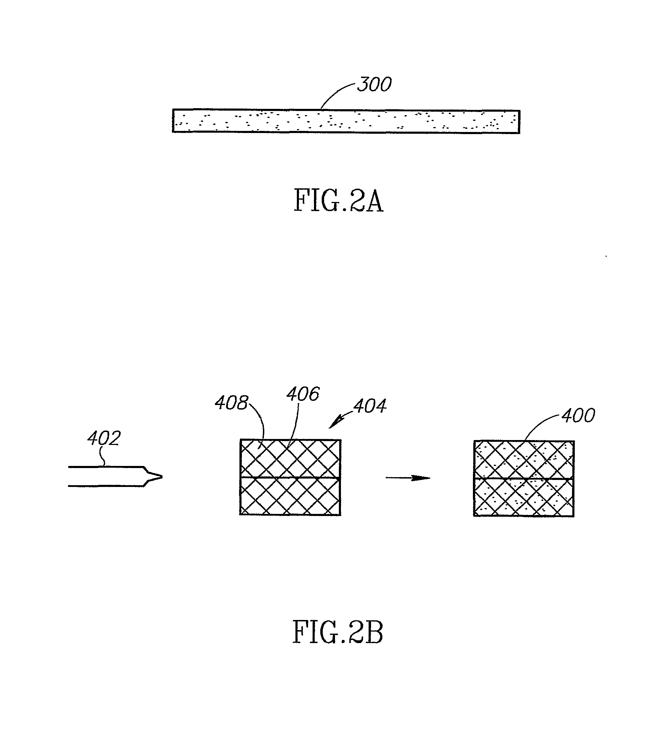

The resulting viscous colloid liquid is then formed (step 116) into a film. This step may include formation of viscous colloid liquid into a free-standing film, as shown in FIG. 2A, or may include formation of a fabric/film composite, as shown in FIG. 2B. In one embodiment, a free-standing film 300, as shown in FIG. 2A, is formed as follows. First, measured amounts of the viscous liquid are poured into film molds and dried under vacuum in a vacuum oven at a relatively low temperature (approximately 20°-30° C) to create a film. Once the film is dried, the temperature may be raised to 100°-110° C and heated for a period of time (approximately 24 hours) under vacuum. This last step, if performed properly, can simultaneously enhance the natural cross-linking of the collagen, providing greater stability, while also sterilizing the film. If done for too long or at too high a temperature, the collagen may start to denature. In other embodiments, the viscous liquid may be directly formed into a film using adapted sheeting methods. In some embodiments, following film formation, the films or samples of the films may be tested (step 118) for fibroblast proliferation.

Films may vary in thickness from 25 to 100 microns. The more spread out the gel is, the thinner the film will be. In some embodiments, small amounts of food grade agents may be added to enhance film pliability and/or stability. In addition, any enhancement agents, such as vitamin C or other natural healing elements may be added. Films may be tested for fibroblast attachment and proliferation in vitro, and may be used for wound dressings and other applications.

In a second embodiment, a film/fabric composite 400 is formed either by immersing a piece of fabric 404 in the gel, or by injecting the viscous colloid liquid via an injection device 402 such as syringe into the piece of fabric 404. Fabric 404 may be gauze, woven fabric, or any other suitable fabric. In some embodiments, fabric 404 is comprised of structural elements 406 and pores 408. Structural elements 406 may have a rope-like structure, a weave-like structure, or any other structure which may allow for the presence of pores 408. After injection, the film/fabric composite may be dried under vacuum in a vacuum oven at a relatively low temperature (approximately 20°-30° C). Once the film is dried, the temperature may be raised to 100°-110° C and heated for a period of time (approximately 24 hours) under vacuum.

An advantage of this embodiment is that pores are naturally present in the material, which can enhance exudation and healing of the wound. In a free-standing film, as in the first embodiment, it may be necessary to introduce pores, which can be done, for example, by deaerating and then injecting air into the gel at a fixed rate and speed, or by using lasers.

In some embodiments, small amounts of food grade agents may be added to enhance film pliability and/or stability. In addition, any enhancement agents, such as vitamin C or other natural healing elements may be added. Film/fabric composites may be tested for fibroblast attachment and proliferation in vitro, and may be used for wound dressings and other applications.

## Claims

1. A method of producing a collagen-based gel, the method comprising: providing a jellyfish tissue;
desalting said jellyfish tissue;
chopping said jellyfish tissue;
determining an isoelectric point of said jellyfish tissue;
adding an acid of a concentration and molarity which is dependent on an isoelectric point of said jellyfish tissue to said jellyfish tissue to produce a collagen-salt colloid; and
mixing said collagen-salt colloid to form a viscous gel.

2. The method of claim 1, further comprising forming a film or a film/fabric composite from said gel.

3. The method of claim 1, wherein said adding an acid comprises adding a strong acid in a range of 0.001 and 0.1 M concentration for a 1-3% solution of jellyfish tissue.

4. The method of claim 1 further comprising monitoring a pH during said adding an acid.

5. The method of claim 1, wherein said mixing comprises at least one of: homogenizing, stirring, blending, agitating or any combination thereof.

6. The method of claim 1, wherein during said mixing, measurements of viscosity are taken.

7. The method of claim 2, further comprising drying the film for a period of time under dehumidified air or vacuum, preferably wherein said drying further includes sterilizing the film.

8. The method of claim 2, further comprising testing the created film for fibroblast proliferation.

9. The method of any of the preceding claims further comprising forming a wound dressing from said gel.

10. A wound dressing comprising: colloidal collagen-based gel obtainable by the method of claim 9 from jellyfish tissue configured to be placed on a wound.

11. The wound dressing of claim 10, wherein said colloidal collagen-based gel is a film or a film/fabric composite, preferably wherein said film/fabric composite has a porous structure.

12. The wound dressing of claim 10 or the method of claim 1, wherein said jellyfish tissue comprises Rhopilema nomadica jellyfish tissue.

13. The wound dressing of claim 10, further comprising food grade agents.

14. The wound dressing of claim 11, wherein said film is sterilized during a process of film formation.

15. The wound dressing of claim 11, wherein said film has a thickness in a range of 25 to 100 microns.

## Patentansprüche

1. Verfahren zur Herstellung eines Kollagen-basierten Gels, wobei das Verfahren Folgendes umfasst: die Bereitstellung eines Quallengewebes;
die Entsalzung des Quallengewebes;
das Zerhacken des Quallengewebes;
die Bestimmung eines isoelektrischen Punkts des Quallengewebes;
die Hinzufügung einer Säure mit einer Konzentration und Molarität, die von einem isoelektrischen Punkt des Quallengewebes abhängig ist, zu dem Quallengewebe, um ein Kollagen-Salz-Kolloid herzustellen; und
das Vermischen des Kollagen-Salz-Kolloids, um ein viskoses Gel zu erzeugen.

2. Verfahren nach Anspruch 1, das zusätzlich die Herstellung eines Films oder eines Verbundfilms/Verbundstoffes aus dem Gel umfasst.

3. Verfahren nach Anspruch 1, wobei die Hinzufügung einer Säure die Hinzufügung einer starken Säure in einem Konzentrationsbereich von 0,001 und 0,1 M für eine 1-3 %-ige Lösung eines Quallengewebes umfasst.

4. Verfahren nach Anspruch 1, das zusätzlich die Überwachung eines pH-Werts während der Hinzufügung einer Säure umfasst.

5. Verfahren nach Anspruch 1, wobei das Vermischen mindestens eines der Folgenden umfasst: Homogenisieren, Rühren, Vermischung, Nachmischen oder eine beliebige Kombination davon.

6. Verfahren nach Anspruch 1, wobei während des Vermischens Messungen hinsichtlich der Viskosität vorgenommen werden.

7. Verfahren nach Anspruch 2, das zusätzlich das Trocknen des Films über einen bestimmten Zeitraum bei entfeuchteter Luft oder im Vakuum umfasst, vorzugsweise wobei das Trocken zusätzlich die Sterilisation des Films umfasst.

8. Verfahren nach Anspruch 2, wobei der erzeugte Film zusätzlich auf Fibroblastenproliferation geprüft wird.

9. Verfahren nach einem beliebigen der vorhergehenden Ansprüche, das zusätzlich die Herstellung einer Wundauflage aus dem Gel umfasst.

10. Wundauflage, die Folgendes umfasst: ein kolloidales Kollagen-basiertes Gel, das durch das Verfahren nach Anspruch 9 aus Quallengewebe hergestellt werden kann und so konfiguriert ist, um auf eine Wunde gelegt zu werden.

11. Wundauflage nach Anspruch 10, wobei das kolloidale Kollagen-basierte Gel ein Film oder ein Verbundfilm/Verbundstoff ist, vorzugsweise wobei der Verbundfilm/Verbundstoff eine poröse Struktur aufweist.

12. Wundauflage nach Anspruch 10 oder Verfahren nach Anspruch 1, wobei das Quallengewebe Rhopilema-nomadica-Quallengewebe enthält.

13. Wundauflage nach Anspruch 10, die zusätzlich lebensmitteltaugliche Stoffe enthält.

14. Wundauflage nach Anspruch 11, wobei der Film während eines Verfahrens zur Filmbildung sterilisiert wird.

15. Wundauflage nach Anspruch 11, wobei der Film eine Dicke in einem Bereich von 25 bis 100 Mikrometer aufweist.

## Revendications

1. Procédé de fabrication d'un gel à base de collagène, le procédé comprenant les phases suivantes:
fourniture d'un tissu de méduse ;
désalinisation dudit tissu de méduse ;
hâchage dudit tissu de méduse ;
détermination d'un point isoélectrique dudit tissu de méduse ;
addition d'un acide ayant une concentration et une molarité liées à un point isoélectrique dudit tissu de méduse, audit tissu de méduse pour produire un colloïde de sel collagène; et
mélange dudit colloïde de sel collagène pour constituer un gel visqueux.

2. Procédé selon la revendication 1, consistant en outre à former un film ou bien un composite de film et de tissu à partir dudit gel.

3. Procédé selon la revendication 1, où ladite addition d'un acide consiste à ajouter un fort acide avec une concentration dans une plage comprise entre 0,001 et 0,1 M pour une solution à 1-3 % de tissu de méduse.

4. Procédé selon la revendication 1, consistant en outre à surveiller un pH pendant ladite addition d'un acide.

5. Procédé selon la revendication 1, où ledit mélange comprend au moins l'une des opérations suivantes : homogénéisation, brassage, malaxage, agitation ou toute combinaison de ceux-ci.

6. Procédé selon la revendication 1, où l'on effectue des mesures de viscosité pendant ledit mélange.

7. Procédé selon la revendication 2, consistant en outre à sécher le film pendant un laps de temps donné sous air déshumidifié ou sous vide, de préférence lorsque ledit séchage consiste en outre à stériliser le film.

8. Procédé selon la revendication 2, consistant en outre à rechercher la prolifération des fibroblastes dans le film ainsi obtenu.

9. Procédé selon l'une quelconque des revendications précédentes, consistant en outre à former un pansement à partir dudit gel.

10. Pansement comprenant : un gel à base de collagène colloïdal que l'on peut obtenir avec le procédé de la revendication 9 à partir de tissu de méduse configuré pour être appliqué sur une blessure.

11. Pansement de la revendication 10, où ledit gel à base de collagène colloïdal est un film ou un composite de film et de tissu, de préférence où ledit composite de film et de tissu a une structure poreuse.

12. Pansement de la revendication 10 ou procédé de la revendication 1, où ledit tissu de méduse comprend du tissu de méduse Rhopilema nomadica.

13. Pansement de la revendication 10, comprenant en outre des agents de qualité alimentaire.

14. Pansement de la revendication 11, où ledit film est stérilisé pendant un processus de formation de film.

15. Pansement de la revendication 11, où ledit film a une épaisseur comprise entre 25 et 100 microns.
